# EUROPÄISCHE PATENTANMELDUNG

(11) **EP 1 043 017 A2**
(43) Veröffentlichungstag der Anmeldung: **11.10.2000**
(21) Anmeldenummer: 00106912.9
(22) Anmeldetag: 31.03.2000
(51) Int. Cl.: A61K 7/48

(54) **Ceramide enthaltendes Hautpflegemittel**

(30) Priorität: 09.04.1999 DE 19916090
(71) Anmelder: Clariant GmbH, 65929 Frankfurt am Main (DE)
(72) Erfinder: Glauder, Jan, Dr., 65929 Frankfurt (DE); Löffler, Matthias, Dr., 65527 Niedernhausen (DE); Henning, Torsten, Dr., 65779 Kelkheim (DE); Turowski-Wanke, Angelika, Dr., 65779 Kelkheim (DE)

(57) **Zusammenfassung**

Es werden Hautpflegemittel beansprucht, enthaltend Fettsäure-N-alkylpolyhydroxyalkylamide der Formel (I) 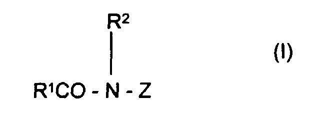 wobei R¹ einen linearen oder verzweigten, gesättigten oder ungesättigten Alkylrest mit 6 bis 22 Kohlenstoffatomen, R² Wasserstoff oder einen Alkyl- oder Hydroxyalkylrest mit 1 bis 3 Kohlenstoffatomen und Z einen linearen oder verzweigten Polyhydroxyalkylrest mit 3 bis 12 Kohlenstoffatomen und 3 bis 10 Hydroxylgruppen bedeutet.

## Beschreibung

Ceramide sind in der menschlichen Haut der Hauptbestandteil des Stratum corneum. Gemeinsam mit den neutralen Lipiden bilden sie breite geschichtete interzelluläre Plättchen und spielen eine wesentliche Rolle in der Haut-Barriere-Funktion. Diese interzellulären Lipide sind hauptsächlich verantwortlich für die Wasserrückhaltung im Stratum corneum, wobei die Hautceramide eine spezielle Rolle spielen. Daher können ceramidhaltige Hautkosmetika einen wesentlichen Beitrag zur Wiederherstellung einer gestörten Haut-Barriere-Funktion leisten. Ceramide stellen lipophile Amide dar, basierend auf Sphingosin bzw. Phytosphingosin, verknüpft mit langkettigen Fettsäuren.

Dem Einsatz von Ceramiden in Hautpflegemitteln sind infolge ihrer mangelnden Verfügbarkeit, aber auch wegen ihrer mangelhaften Löslichkeit in Hautpflegemittelbestandteilen bislang Grenzen gesetzt. Man ist daher bestrebt, ceramidanaloge Strukturen, sogenannte "synthetic barrier lipids (SBL)" oder "Pseudoceramide" zu synthetisieren und zur Hautpflege einzusetzen (G. Imokawa et al., J. Soc. Cosmet. Chem. 40, 273 (1989)).

Ceramidverwandte Verbindungen sind bereits in EP-A-0 277 641, EP-A-0 227 994 und EP-A-0 495 624 beschrieben.

Für den Schutz von Haut und Haaren werden in EP-A-0 455 429 Zuckerderivate der folgenden Verbindung vorgeschlagen:

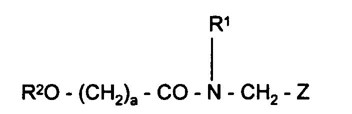

Hierbei steht R¹ für Wasserstoff oder einen ungesättigten Fettacylrest, a für Zahlen von 7 bis 49, R² für einen Hydroxyalkylrest und Z für einen Zucker- oder Phosphatrest.

In WO 95/05152 werden Fettsäure-N-alkylpolyhydroxyalkylamide der Formel 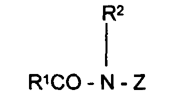 als "synthetic barrier lipids" beschrieben, wobei R¹ für einen aliphatischen Alkylrest mit 6 bis 22 Kohlenstoffatomen, R² einen Alkyl- oder Hydroxyalkylrest mit 4 bis 22 Kohlenstoffatomen und Z für einen linearen oder verzweigten Polyhydroxyalkylrest mit 3 bis 12 Kohlenstoffatomen und 3 bis 10 Hydroxylgruppen steht.

Alle bislang beschriebenen Pseudoceramide sind in ihrem Leistungsvermögen bezüglich der Restaurierung der Lipidbarriere unbefriedigend. Die Aufgabe der Erfindung bestand darin, neue leistungsstarke ceramidanaloge Strukturen zu entwickeln, die zudem einfach zu synthetisieren sind und anwendungstechnisch gut zu handhaben sind.

Es wurde nun gefunden, daß sich auch solche Fettsäure-N-alkylpolyhydroxyalkylamide der obigen Formel als Wirkstoff in Hautpflegemitteln eignen, die eine N-Alkylgruppe mit nur 1 bis 3 C-Atomen enthalten.

Gegenstand der Erfindung sind Hautpflegemittel, enthaltend Fettsäure-N-alkylpolyhydroxyalkylamide der Formel I 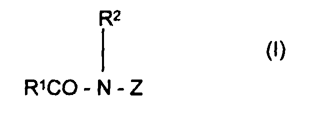 wobei R¹ einen linearen oder verzweigten, gesättigten oder ungesättigten Alkylrest mit 6 bis 22, vorzugsweise 12 bis 18 Kohlenstoffatomen, R² Wasserstoff oder einen Alkyl- oder Hydroxyalkylrest mit 1 bis 3 Kohlenstoffatomen und Z einen linearen oder verzweigten Polyhydroxyalkylrest mit 3 bis 12 Kohlenstoffatomen und 3 bis 10 Hydroxylgruppen bedeutet.

Die Fettsäure-N-alkylpolyhydroxyalkylamide der genannten Formel werden durch reduktive Aminierung eines reduzierenden Zuckers mit einem Amin oder einem Alkanolamin und nachfolgende Acylierung mit einer Fettsäure, einem Fettsäurealkylester oder vorzugsweise einem Fettsäurechlorid in Gegenwart einer Base (Triethanolamin, Natriumcarbonat etc.) erhalten. Hinsichtlich der Einzelheiten dieses Verfahrens sei auf die US-Patentschriften US 1 985 424, US 2 016 962 und US 2 703 798 sowie die Internationale Patentanmeldung WO 92/06984 verwiesen.

Vorzugsweise leiten sich die Polyhydroxyfettsäureamide von reduzierenden Zuckern mit 5 oder 6 Kohlenstoffatomen ab. Der Rest Z ist daher beispielsweise ein 1-Deoxyglucityl-, 2-Deoxyfructityl-, 1-Deoxymaltityl-, 1-Deoxylactityl-, 1-Deoxygalactityl-, 1-Deoxymannityl-, 1-Deoxymaltotriotityl-Rest. Der Rest R¹CO-steht bevorzugt für den Acylrest der Cocosfettsäure, Stearinsäure, Isostearinsäure, Ölsäure, Elaidinsäure, Petroselinsäure, Laurinsäure, Myristinsäure, Caprinsäure, Palmitinsäure oder Talgfettsäure. R¹ ist vorzugsweise Methyl, besonders bevorzugt sind C₈-C₂₂-, insbesondere C₁₂-C₁₈-Acyl-N-methylglucamide.

Die erfindungsgemäßen Hautpflegemittel können die Fettsäure-N-alkylpolyhydroxyalkylamide in Mengen von 0,1 bis 30 Gew.-%, vorzugsweise von 0,5 bis 15 Gew.-%, besonders bevorzugt von 1 bis 5 Gew.-%, bezogen auf das gesamte Hautpflegemittel, enthalten.

Die erfindungsgemäßen Mittel können als "Wasser-in-Öl" oder "Öl-in-Wasser-Emulsionen" vorliegen und dabei weitere übliche Hilfs- und Zusatzstoffe in Mengen von 5 bis 95 Gew.-%, vorzugsweise 10 bis 80 Gew.-% aufweisen. Ferner können die Formulierungen Wasser in einer Menge bis zu 99 Gew.-%, vorzugsweise 5 bis 80 Gew.-%, enthalten.

Als Trägeröle kommen beispielsweise Guerbetalkohole mit 6 bis 18, vorzugsweise 8 bis 10 Kohlenstoffatomen, Ester von linearen C₆-C₁₃-Fettsäuren mit linearen C₆-C₂₀-Fettalkoholen, Ester von verzweigten C₆-C₁₃-Carbonsäuren mit linearen C₆-C₂₀-Fettalkoholen, Ester von linearen C₆-C₁₈-Fettsäuren mit verzweigten Alkoholen, insbesondere 2-Ethylhexanol, Ester von linearen und/oder verzweigten Fettsäuren mit mehrwertigen Alkoholen (wie z.B. Dimerdiol oder Trimerdiol) und/oder Guerbetalkoholen, Triglyceride auf Basis C₆-C₁₀-Fettsäuren, pflanzliche Öle, Mineralöle, Siliconöle, verzweigte Alkohole, substituierte Cyclohexane, Guerbetcarbonate, Dialkylether und/oder alipahtische bzw. aromatische Kohlenwasserstoffe in Betracht.

Die erfindungsgemäßen Hautpflegemittel, wie beispielsweise Tagescremes, Nachtcremes, Pflegecremes, Nährcremes, Sonnenschutzcremes, Rasiercremes, Bodylotions, Hautöle, Deodorant-Stifte, Körper-Aerosole und Salben können als weitere Hilfs- und Zusatzstoffe Emulgatoren, Co-Emulgatoren, Überfettungsmittel, Fette, Wachse, Stabilisatoren, biogene Wirkstoffe, Glycerin, Konservierungsmittel, Perlglanzmittel, Farb- und Duftstoffe enthalten.

Als Emulgatoren können beispielsweise eingesetzt werden: Sorbitanester, Sorbitolester, Phosphorsäureester, Monoglyceride, Polysorbate, Polyethlyenglycolmono/difettsäureester, hochethoxylierte Fettsäureester sowie hochmolekulare Siliconverbindungen, wie z.B. Dimethylpolysiloxane mit einem durchschnittlichen Molekulargewicht von 10.000 bis 50.000.

Als nichtionogene O/W-Co-Emulgatoren kommen in Betracht Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe; C₁₂-C₁₈-Fettsäuremono- und -diester von Anlagerungsprodukten von 1 bis 30 Mol Ethylenoxid an Glycerin, Glycerinmono- und -diester und Sorbitanmono- und -diester von gesättigten und ungesättigten Fettsäuren mit 6 bis 22 Kohlenstoffatomen und deren Ethylenoxidanlagerungsprodukten; Anlagerungsprodukte von 15 bis 60 Mol Ethylenoxid an Rizinusöl und/oder gehärtetes Rizinusöl, Polyol- und insbesondere Polyglycerinester, wie z.B. Polyglycerinpolyricinoleat und Polyglycerinpoly-12-hydroxystearat. Ebenfalls geeignet sind Gemische von Verbindungen aus mehreren dieser Substanzklassen. Die Anlagerungsprodukte von Ethylenoxid und/oder Propylenoxid an Fettalkohole, Fettsäuren, Alkylphenole, Glycerin-mono- und-diester sowie Soribtanmono- und -diester oder Sorbitolmono- und -diester von Fettsäuren oder an Rizinusöl steilen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht. C₁₂-C₁₈-Fettsäuremono- und -diester von Anlagerungsprodukten von Ethylenoxid an Glycerin sind aus DE 20 24 051 als Rückfettungsmittel für kosmetische Zubereitungen bekannt.

Als Überfettungsmittel können Substanzen wie beispielsweise polyethoxylierte Lanolinderivate, Lecithinderivate, Polyolfettsäureester, Monoglyceride und Fettsäurealkanolamide verwendet werden, wobei die letzteren gleichzeitig als Schaumstabilisatoren dienen. Typische Beispiele für Fette sind Glyceride, als Wachse kommen u.a. Bienenwachs, Paraffinwachs oder Mikrowachse, gegebenenfalls in Kombination mit hydrophilen Wachsen, z.B. Cetylstearylalkohol in Frage.
Als Stabilisatoren können Metallsalze von Fettsäuren, wie z.B. Magnesium-, Aluminium- und/oder Zinkstearat eingesetzt werden.
Unter biogenen Wirkstoffen sind beispielsweise Pflanzenextrakte und Vitaminkomplexe zu verstehen.
Als Konservierungsmittel eignen sich beispielsweise Phenoxyethanol, Formaldehydlösung, Parabene, Pentandiol oder Sorbinsäure. Als Perlglanzmittel kommen beispielsweise Glycoldistearinsäureester, wie Ethylenglycoldistearat, aber auch Fettsäuremonoglycolester in Betracht.
Als Farbstoffe können die für kosmetische Zwecke geeigneten und zugelassenen Substanzen verwendet werden, wie sie beispielsweise in der Publikation "Kosmetische Färbemittel" der Farbstoffkommission der Deutschen Forschungsgemeinschaft, Verlag Chemie, Weinheim, 1984, S. 81 - 106 zusammengestellt sind.

Der Gesamtanteil der Hilfs- und Zusatzstoffe kann 1 bis 10 ew.-%, vorzugsweise 2 bis 5 Gew.-%, bezogen auf das Mittel, betragen.

Die Fettsäure-N-alkylpolyhydroxyalkylamide gemäß der Formel I können auch mit konventionellen Ceramiden, Pseudoceramiden, Cholesterin, Cholesterinfettsäureestern, Fettsäuren, Triglyceriden, Cerebrosiden, Phospholipiden und ähnlichen Stoffen, abgemischt werden, wobei Liposomen entstehen können.

In einer weiteren Ausführungsform der Erfindung können die Fettsäure-N-alkylpolyhydroxyalkylamide mit Wirkstoffbeschleunigern, insbesondere mit etherischen Ölen, wie beispielsweise Eucalyptol, Menthol und ähnlichen abgemischt werden.

Darüberhinaus können die Fettsäure-N-alkylpolyhydroxyalkylamide auch in Squalen oder Squalan gelöst und gegebenenfalls mit den anderen genannten Inhaltsstoffen zusammen mit flüchtigen oder nichtflüchtigen Siliconverbindungen als wasserfreie oder beinahe wasserfreie einphasige Systeme formuliert werden.

### Beispiele

### O/W-Handpflegecreme

| | |
|---|---|
| Hostaphat KW 340 | 3,0 % |
| Cetylstearylalkohol | 9,0 % |
| Glycerinmonostearat | 3,0 % |
| Decyloleat | 6,0 % |
| Paraffinöl, perliquidum | 4,0 % |
| Glycerin | 5,0 % |
| Lauroyl-N-methylglucamid | 6,0 % |
| Mandelproteinhydrolysat | 5,0 % |
| Konservierungsmittel | q.s. |
| Wasser | ad 100 % |

### Herstellung

Hostaphat KW 340, Cetylstearylalkohol, Glycerinmonostearat, Decyloleat, Paraffinöl wurden bei ca. 80°C aufgeschmolzen.
Glycerin, Lauroyl-N-methylglucamid, Mandelhydrolysat und Wasser wurden auf ca. 80°C erwärmt (homogene Lösung) und unter Rühren der Emulgator-Fettphase zugesetzt und kaltgerührt. Bei ca. 40°C Konservierungsmittel eingerührt und abschließend die Emulsion homogenisiert.

### O/W-Softcreme

| | |
|---|---|
| Hostacerin DGMS | 5,0 % |
| 2-Octyldodecanol | 3,0 % |
| Decyloleat | 5,0 % |
| Dimethicone (Abil 100) | 0,5 % |
| Paraffin, subliquidum | 8,0 % |
| Carbomer (Carbopol 980) | 0,30 % |
| Glycerin | 3,0 % |
| Lauroyl-N-methylglucamid | 2,0 % |
| NaOH (10 % in Wasser) | 1,1 % |
| Hostapon KCG | 0,60 % |
| Konservierungsmittel | q.s. |
| Wasser | ad 100% |

### Herstellung

Hostacerin DGMS, 2-Octyldodecanol, Decyloleat, Dimethicone, Paraffinöl wurden bei ca. 80°C aufgeschmolzen, anschließend wurde Carbomer zugegeben.Die 80°C heiße Lösung aus Glycerin, NaOH, Hostapon KCG, Lauroyl-N-methylglucamid, Wasser wurde unter Rühren der Emulgator-Fettschmelze zugesetzt und kaltgerührt. Bei ca. 40°C wurde das Konservierungsmittel eingerührt und abschließend die Emulsion homogenisiert.

### O/W-Nähr- und Pflegecreme

| | |
|---|---|
| Hostaphat KW 340 | 3,0 % |
| Palmitin/stearinsäure | 3,0 % |
| Cetylstearylalkohol | 3,0 % |
| 2-Octyldodecanol | 7,0 % |
| Paraffinöl, perliquidum | 4,0 % |
| Glycerin | 5,0 % |
| C₁₆/C₁₈-Acyl-N-methylglucamid | 5,0 % |
| KOH (20 % in Wasser) | 2,0 % |
| Keratinhydrolysat (20 %ig) | 5,0 % |
| Konservierungsmittel | q.s. |
| Wasser | ad 100% |

### Herstellung

Hostaphat KW 340, Palmitin/Stearinsäure, Cetylalkohol, 2-Octyldocecanol, Paraffinöl wurden bei ca. 80°C aufgeschmolzen. Die ca. 80°C heiße Lösung aus Glycerin, Acyl-N-methylglucamid, KOH, Wasser wurde unter Rühren der Emulgator-Fettphase zugesetzt und kaltgerührt. Bei ca. 40°C wurden das Keratinhydrolysat und das Konservierungsmittel eingerührt, abschließend die Emulsion homogenisiert.

### O/W-Hautcreme

| | |
|---|---|
| Hostacerin DGMS | 4,0 % |
| Cetylalkohol | 1,0 % |
| Glycerinmonostearat | 2,0 % |
| Stearinsäure | 1,5 % |
| Lauroyl-N-methylglucamid | 6,0 % |
| Decyloleat | 3,0 % |
| Cetearylisononanoat | 3,0 % |
| Paraffin, subliquidum | 4,0 % |
| Glycerin | 5,0 % |
| Konservierungsmittel | q.s. |
| Wasser | ad 100 % |

### Herstellung

Hostacerin DGMS, Cetylalkohol, Glycerinmonostearat, Stearinsäure, Decyloleat, Cetearylisononanoat und Paraffin wurden auf ca. 80°C erhitzt. Dieser heißen Schmelze wurde eine auf ca. 80°C erhitzte Lösung aus Lauroyl-N-methylglucamid, Glycerin und Wasser zugegeben und die Mischung wurde kaltgerührt. Bei ca. 40°C wurde das Konservierungsmittel zugegeben und die Emulsion wurde abschließend kaltgerührt.

### O/W-Hautcreme

| | |
|---|---|
| Hostacerin DGl | 2,0 % |
| Paraffinöl, perliquidum | 8,0 % |
| Octyldodecanol | 4,0 % |
| Carbomer (Carbopol 980) | 0,7 % |
| C₁₆/C₁₈-Acyl-N-methylglucamid | 5,0 % |
| Hostapon KCG | 0,6 % |
| NaOH (10 %ig in Wasser) | 2,4 % |
| Konservierungsmittel | q.s. |
| Wasser | ad 100 % |

### Herstellung

Hostacerin DGl, Paraffinöl, Octyldodecanol wurden auf ca. 80°C erhitzt, anschließend wurde Carbomer zugesetzt. Die ca. 80°C heiße Lösung aus Acyl-N-methylglucamid, Hostapon KCG, NaOH und Wasser wurde der Emulgator-Fettschmelze zugesetzt und kaltgerührt. Bei ca. 40°C wurde das Konservierungsmittel zugesetzt, anschließend die Emulsion homogenisiert.

### O/W-Sonnenschutzmilch

| | |
|---|---|
| Hostacerin DGL | 1,0 % |
| Hostacerin DGSB | 4,0 % |
| Paraffinöl, subliquidum | 6,0 % |
| Avocadoöl | 1,0 % |
| Dimethicone (Abil 100) | 1,0 % |
| Isoamyl-p-Methoxycinnamat (Neo-Heliopan E-1000) | 9,0 % |
| Benzophenone-3 (Neo-Heliopan BB) | 1,0 % |
| Carbopol 980 | 0,4 % |
| Lauroyl-N-methylglucamid | 5,0 % |
| Ethylendiamin Tetraacetic Acid Sodium Salt (Aquamollin BC Plv. Hochkonz.) | 0,1 % |
| Citronensäure (10 % in Wasser) | 0,3 % |
| NaOH (10 %ig in Wasser) | 1,5 % |
| Konservierungsmittel | q.s. |
| Wasser | ad 100 % |

### Herstellung

Hostacerin DGL, Hostacerin DGSB, Paraffinöl, Avocadoöl, Dimethicone, Isoamyl p-Methoxicinnamate, Benzophenone-3 wurden bei ca. 80°C aufgeschmolzen, anschließend wurde Carbomer zugesetzt. Die ca. 80°C heiße Lösung aus Lauroyl-N-methylglucamid, Ethylendiamin Tetraacetic Acid Sodium Salt, Citronensäure, NaOH, Wasser wurde unter Rühren der Emulgator-Fettschmelze zugesetzt und kaltgerührt. Bei ca. 40°C wurde das Konservierungsmittel eingerührt, abschließend die Emulsion homogenisiert.

### W/O-Pflegecreme

| | |
|---|---|
| Hostacerin WO | 10,0 % |
| Bienenwachs | 2,0 % |
| Paraffinöl, subliquidum | 8,0 % |
| Sonnenblumenöl | 4,0 % |
| Weizenkeimöl | 1,0 % |
| Avocadoöl | 3,0 % |
| Isopropylisostearat | 4,0 % |
| Lauroyl-N-methylglucamid | 4,0 % |
| Glycerin | 5,0 % |
| Ethylendiamin Tetraacetic Acid Sodium Salt (Aquamollin BC Plv. Hochkonz.) | 0,1 % |
| Citronensäure (10 % in Wasser) | 0,3 % |
| MgSO₄ * 7 H₂O | 0,7 % |
| Konservierungsmittel | q.s. |
| Wasser | ad 100 % |

### Herstellung

Hostacerin WO, Bienenwachs, Paraffinöl, Sonnenblumenöl, Weizenkeimöl, Avocadoöl, Isopropylstearat wurden bei ca. 80°C aufgeschmolzen. Die ca. 80°C heiße Lösung aus Lauroyl-N-methylglucamid, Glycerin, Ethylendiamin, Tetraacetic Acid Sodium Salt, Citronensäure, Magnesiumsulfat und Wasser wurde unter Rühren der Emulgator-Fettschmelze zugesetzt und kaltgerührt. Bei ca. 40°C wurde das Konservierungsmittel eingerührt.

### W/O-Nachtcreme

| | |
|---|---|
| Hostacerin DGl | 4,0 % |
| PEG 7 hydrogenated Castor Oil | 3,0 % |
| Cetylstearylalkohol | 1,0 % |
| Bienenwachs | 5,0 % |
| Zinkstearat | 0,5 % |
| Paraffinöl, perliquidum | 7,0 % |
| Octyl Stearate | 6,0 % |
| C₁₆/C₁₈-Acyl-N-methylglucamid | 7,0 % |
| Glycerin | 5,0 % |
| MgSO₄ * 7 H₂O | 0,7 % |
| Mandelproteinhydrolysat (22 %ig in Wasser) | 2,5 % |
| Konservierungsmittel | q.s. |
| Wasser | ad 100 % |

### Herstellung

Hostacerin DGl, PEG-7 hydrogenated Castor Oil, Cetylstearylalkohol, Bienenwachs, Zinkstearat, Paraffinöl, Octyl Stearate wurden bei ca. 80°C aufgeschmolzen (Bemerkung: Zinkstearat wird hier nur angeschmolzen, die Fettphase wird als nicht klar). Die ca. 80°C heiße Lösung aus Acyl-N-methylglucamid, Glycerin, Magnesiumsulfat, Wasser wurde unter Rühren der Emulgator-Fettschmelze zugesetzt und kaltgerührt. Bei ca. 50°C wurde das Mandelhydrolysat und bei ca. 40°C das Konservierungsmittel eingerührt.

### W/O-Pflegelotion

| | |
|---|---|
| Hostacerin WO | 2,0 % |
| PEG 7 Hydrogenated Castor Oil | 2,0 % |
| Paraffinöl, perliquidum | 15,0 % |
| Octyldodecanol | 5,0 % |
| Isopropylpalmitat | 5,0 % |
| C₁₆/C₁₈-Acyl-N-methylglucamid | 6,0 % |
| Glycerin | 4,0 % |
| NaCl | 2,0 % |
| Konservierungsmittel | q.s. |
| Wasser | ad 100 % |

### Herstellung

Hostacerin WO, PEG-7 Hydrogenated Castor Oil, Paraffinöl, Octyldodecanol, Isopropylpalmitat wurden bei ca. 80°C aufgeschmolzen. Die ca. 80°C heiße Lösung aus Acyl-N-methylglucamid, Glycerin, NaCl, Wasser wurde unter Rühren der Emulgator-Fettschmelze zugesetzt und kaltgerührt. Bei ca. 40°C wurde das Konservierungsmittel eingerührt.

### Chemische Bezeichnung der eingesetzten Handelsprodukte

| | |
|---|---|
| Hostaphat KW 340 | Mono-, Di- und Tri(cetearyltetraglycolether)-o-Phosphorsäureester (Clariant GmbH) |
| Hostapon KCG | Natriumcocoylglutamat (Clariant GmbH) |
| Hostacerin DGMS | Polyglyceryl-2-Stearat (Clariant GmbH) |
| Hostacerin DGl | Polyglyceryl-2-Sesquiisostearat (Clariant GmbH) |
| Hostacerin DGL | PEG-10 Polyglyceryl-2 Laurat (Clariant GmbH) |
| Hostacerin DGSB | PEG-4 Polyglyceryl-2 Stearat (Clariant GmbH) |
| Hostacerin WO | Polyglyceryl-2 Sesquiisostearat, (Clariant GmbH) |
| | Bienenwachs, Mineralöl |
| | Magnesiumstearat, Aluminium-Stearat |
| Carbopol 980 | Carbomer (Goodrich) |
| Dimethicone | Dimethylsiloxan |

## Patentansprüche

1. Hautpflegemittel, enthaltend Fettsäure-N-alkylpolyhydroxyalkylamide der Formel (I) 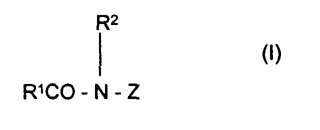 wobei R¹ einen linearen oder verzweigten, gesättigten oder ungesättigten Alkylrest mit 6 bis 22 Kohlenstoffatomen, R² Wasserstoff oder einen Alkyl- oder Hydroxyalkylrest mit 1 bis 3 Kohlenstoffatomen und Z einen linearen oder verzweigten Polyhydroxyalkylrest mit 3 bis 12 Kohlenstoffatomen und 3 bis 10 Hydroxylgruppen bedeutet.

2. Hautpflegemittel nach Anspruch 1, dadurch gekennzeichnet, dass sie Fettsäure-N-methylglucamide enthalten.

3. Hautpflegemittel nach den Ansprüchen 1 und 2, dadurch gekennzeichnet, dass sie Fettsäure-N-methylglucamide der Formel (I) enthalten, in der R¹ einen linearen oder verzweigten, gesättigten oder ungesättigten Alkylrest mit 12 bis 18 Kohlenstoffatomen bedeutet.

4. Hautpflegemittel nach den Ansprüchen 1 bis 3, dadurch gekennzeichnet, dass sie die Fettsäure-N-alkylpolyhydroxyalkylamide in Mengen von 0,1 bis 30 Gew.-%, vorzugsweise von 1 bis 15 Gew.-%, besonders bevorzugt von 2 bis 10 Gew.-%, bezogen auf die gesamte Formulierung, enthalten.
